# EUROPÄISCHE PATENTSCHRIFT

(11) **EP 0 658 119 B1**
(45) Veröffentlichungstag und Bekanntmachung des Hinweises auf die Patenterteilung: **28.04.1999**
(21) Anmeldenummer: 93919158.1
(22) Anmeldetag: 25.08.1993
(51) Int. Cl.: A61K 39/21, C07K 7/04, G01N 33/569, C12N 15/48

(54) **NEUE HIV-1-VIRUSISOLATE EINES SUBTYPS UND SEINE DIFFERENTIELLE DIAGNOSTIK, VAKZINE GEGEN HIV-1-VIRUSINFEKTIONEN DIESES SUBTYPS UND VERFAHREN ZU IHRER HERSTELLUNG, VERWENDUNG DER HIV-1-VIRUSISOLATE**
NEW HIV-1 ISOLATES OF A SUBTYPE AND ITS DIFFERENTIAL DIAGNOSIS, VACCINE AGAINST HIV-1 INFECTION OF THIS SUBTYPE AND PROCESS FOR PREPARING AND USING THE HIV-1 ISOLATE
NOUVEAUX ISOLATS DU VIH 1 D'UN SOUS-TYPE ET SON DIAGNOSTIC DIFFERENTIEL, VACCINS CONTRE LES INFECTIONS A VIH 1 DE CE SOUS-TYPE ET LEUR PROCEDE DE PREPARATION, UTILISATION DES ISOLATS DU VIH 1

(30) Priorität: 29.08.1992 DE 4228787
(43) Veröffentlichungstag der Anmeldung: 21.06.1995
(73) Patentinhaber: CHEMOTHERAPEUTISCHES FORSCHUNGSINSTITUT GEORG-SPEYER-HAUS, D-60596 Frankfurt (DE)
(72) Erfinder: DIETRICH, Ursula, D-65760 Eschborn (DE); VON BRIESEN, Hagen, D-65510 Hünstetten (DE); GREZ, Manuel, D-69221 Dossenheim (DE); RÜBSAMEN-WAIGMANN, Helga, D-65812 Bad Soden (DE)
(74) Vertreter: Werner, Hans-Karsten, Dr.Dipl.-Chem.
(86) Internationale Anmeldenummer: EP9302275
(87) Internationale Veröffentlichungsnummer: WO9405327

(56) Entgegenhaltungen:
- EP-A- 0 327 180
- EP-A- 0 498 905
- WO-A-88/10267
- WO-A-92/05800
- FR-A- 2 657 017
- FR-A- 2 677 364
- US-A- 5 030 449
- US-A- 5 128 319

## Beschreibung

Gegenstand der Erfindung sind neue HIV-1-Virusisolate eines Subtyps, Impfstoffe gegen Virusinfektionen mit diesem HIV-1 Subtyp, Verfahren zu ihrer Herstellung sowie die Verwendung der Virusisolate zur Herstellung von Vakzinen und zur Differentialdiagnostik.

Bisherige Bemühungen, Mittel zur Prophylaxe und Therapie von HIV-Infektionen zu entwickeln, konzentrieren sich auf die Hemmung der reversen Transkriptase oder eines anderen Enzyms des HIV-1 Virus wie zum Beispiel die Protease. Auf diese Weise soll eine spezifische Hemmung der viralen Replikation im Gegensatz zur zellulären Replikation erreicht werden. Trotzdem haben die bisherigen antiviralen Mittel den Nachteil, daß sie mit einer relativ hohen Toxizität für die Zellen verbunden sind, also nicht nur auf das Virus wirken.

Zur Gruppe der Substanzen, bei denen eine Wirkung gegen HIV-Viren festgestllt wurde, gehören unter anderem diverse Nucleosid-Analoga (Azidothymidin, Di-Desoxyinosin und Di-Desoxycytidin). Allerdings wird nach einer gewissen relativ kurzen Zeit der Therapie mit diesen Substanzen eine massive Resistenzbildung des Virus nachgewiesen (Zimmermann et al., abstract no. 3656, IV International Conference on AIDS, Stockholm, 1988, Rübsamen-Waigmann et al., Infection 19, suppl. 2, 77 - 82, 1991). Ferner sind alle genannten Substanzen, zumindest in höheren Dosen, mit erheblichen Nebenwirkungen verbunden.

Ein weitere Hemmstoff der reversen Transkriptase ist die Substanz Suramin.Sie ist jedoch aufgrund ihrer Toxizität für den Säugetierorganismus ebenfalls nicht für eine Prophylaxe oder Therapie von HIV-Virusinfektionen geeignet (H. Mitsuya et al. "Suramin Protection of T-Cells in vitro against Infectivity and Cytopathic Effect of HTLV-III, Science 226 (1984), Seiten 172 - 174).

Die weitere Entwicklung führte dann zu Hemmstoffen der reversen Transkriptase, die weniger toxisch auf den Säugetierorganismus sind. Dazu gehören Stoffe wie Dextrasulfat und Pentosanpolysulfat, die nachweislich in vivo einen inhibierenden Effekt auf HIV-1-Viren zeigen. Diese wird in DE 36 01 136 und EP 0 293 826 beschrieben.

Neben der chemotherapeutischen Behandlung von HIV-Infektionen besteht prinzipiell die Möglichkeit der Gen- oder Immuntherapie. Die Gentherapie umfaßt das Einschleusen von Teilen viraler Nukleinsäuren in menschliche Zellen, insbesondere in die Zielzellen des HIV-Virus, beispielsweise die CD4 positiven Zellen des Immunsystems. Durch Bildung einer "antisense" RNA, d.h. einer zur messenger RNA (m-RNA) komplementären RNA, kann dann die virale m-RNA neutralisiert werden und somit die Weitervermehrung des Virus unterbunden werden. In einer anderen Form können auch direkt Oligonukleotide oder mit ihnen chemisch verwandte Strukturen, die zur m-RNA komplementär sind, eingesetzt werden. Bei der Immuntherapie werden nach der Infektion Antigene gegeben, von denen eine Unterstützung der Immunantwort gegen HIV erwartet wird.

Mittlerweile wird von der Weltgesundheitsorganisation (WHO) eine Zahl von mindestens 13 Millionen HIV-Infizierter weltweit geschätzt; kein Land ist mehr frei von Infektionen mit HIV-1 oder HIV-2 oder beiden Viren gleichzeitig. Um einen weitere Ausbreitung der AIDS Epidemie zu verhindern, ist es dringend erforderlich, einen Impfstoff zum Schutz vor HIV-Infektionen zu entwickeln. Das Hauptproblem hierbei und auch bei der Immuntherapie ist die hohe Variabilität der HIV-Viren: eine Schutzimpfung soll und muß alle möglichen Virusvarianten erfassen. Epidemiologische Untersuchungen verbunden mit der genetischen Charakterisierung der Viren haben ergeben, daß es in beiden Virusfamilien bereits mehrere Subtypen gibt (Myers et al., Human Retroviruses and AIDS, Los Alamos, 1991; Dietrich et al., Nature 342, 948 - 950, 1989), die sich so erheblich in ihrer Erbinformation (und damit auch Antigenität) unterscheiden, daß ein einziger Impfstoff alle Varianten nicht mehr erfassen kann. Darüber hinaus sind diese Subtypen auch geographisch unterschiedlich verteilt.

Die vorliegende Erfindung stellt drei neue HIV-1-Virusisolate, HIV-1_{D757}, HIV-1_{D747}, HIV-1_{D760}, zur Verfügung, die überraschenderweise einen weiteren unabhängigen Subtyp der HIV-1-Familie bilden und aus indischen Patienten gewonnen wurden, die zum Zeitpunkt der Virus-Isolierung keine typischen AIDS-Symptome zeigten.

Die Virusisolate wurden am 27. August 1992 bei der European Collection of Animal Cell Cultures (ECACC), Porton Down, Salisbury, Wiltshire, Großbritannien, gemäß den Bestimmungen des Budapester Vertrages unter folgenden Nummern hinterlegt:
HIV-1_{D747} - ECACC V92082718
HIV-1_{D757} - ECACC V92082719
HIV-1_{D760} - ECACC V92082720

Die Virusisolate vermehren sich auf frischen mononukleären Zellen des peripheren Bluts, also Lymphozyten und Makrophagen.

Des weiteren stellt die Erfindung eine Vakzine mit Wirkungsspektrum gegen diesen spezifischen HIV-1 Subtyp zur Verfügung.

Die Vakzine enthält als Wirkstoff die Peptide
1. MPNGTKSNS, MPNGTKGNS, MPNGTKSNL,
2. RNEKDLLALDSWKN,
Kombinationen mit diesen Peptiden.

Die Peptide leiten sich aus dem env-Bereich der erfindungsgemäßen Stämme ab (siehe Figur 1). Die Nukleotidsequenzen dieser Viren sind im env-Gen über einen Bereich von 1.8 kb nur 79.4 - 81.6 % homolog zu Viren des nordamerikanisch/europäischen Subtyps und 78.9 - 81.2 % homolog zu den Prototyp-Viren des zentralafrikanischen Subtyps. Auf Aminosäureebene beträgt die Homologie jeweils 72.1 - 75.9 % bzw. 71.2 - 74.2 % (siehe Tabelle). Somit stehen diese Viren genetisch zwischen diesen beiden Subtypen und bilden einen weiteren unabhängigen Subtyp, der sich auch von den in Rwanda/Uganda und in Nordthailand gefundene Subtypen durch genetische Äquidistanz abhebt.

Durch Vergleichen der Aminosäuresequenzen der env-Proteine von HIV-1_{D757}, HIV-1_{D747}, HIV-1_{D760}, mit den Aminosäuren-Consensus-Sequenzen, die für alle bereits veröffentlichten HIV-1 env-Sequenzen abgeleitet wurden, ergeben sich für die vorhandenen env-Region 2 kurze Bereiche, in denen sich die drei indischen Sequenzen in 7 von 9 bzw. in 7 von 14 Aminosäuren von allen anderen HIV-1 Sequenzen unterscheiden (Figur 1). Peptide aus diesen Bereichen sind vorzugsweise für einen Impfstoff mit spezifischem Wirkungsspektrum gegen den indischen Subtyp geeignet, besonders, wenn der Impfstoff noch aus einer Kombination dieser Peptide besteht.

Die erfindungsgemäßen Peptide werden synthetisch hergestellt und wenn vorteilhaft, chemisch modifiziert. Diese Peptide oder längere sowie kürzere, bis zu 7 Aminosäuren lange Unterpeptide davon sind ebenso Bestandteil dieser Erfindung wie ihre modifizierten Formen.

Weiterhin ist auch eine Vakzine Gegenstand der Erfindung, die als Wirkstoff Peptide aus variablen Bereichen anderer Gene von HIV-1_{D757}, HIV-1_{D747}, HIV-1_{D760}, enthält, sofern die Abweichung von der entsprechenden Sequenz von HIV-1 Viren anderer Subtypen mehr als 30 % beträgt.

Ferner können in dem Impfstoff auch Antigene aus den erfindungsgemäßen Viren verwendet werden oder Peptid-Kombinationen oder Kombinationen von Peptiden und Antigenen. Insbesondere ist die Verwendung von Antigenen oder Peptiden bevorzugt, die innerhalb der Schleife des Hüllglycoproteins (Aminosäure 423-450 bezogen auf HIV-1_{Lai}), die für die Bindung an den CD4-Rezeptor verantwortlich ist, eine Glycosylierungsstelle (NXT/S) direkt vor dem Cystein 450 (HIV-1_{Lai}), enthalten. Diese Glycosylierungsstelle ist typisch für die indischen Viren HIV-1_{D757}, HIV-1_{D747} und HIV-1_{D760}. Unter 29 weltweit sequenzierten HIV-1 env-Genen tritt diese Glycosylierungsstelle nicht auf.

Eine bevorzugte Ausführungsform der Erfindung ist eine Vakzine, die zum prophylaktischen Einsatz vor der Infektion eingesetzt wird. Eine weitere bevorzugte Ausführungsform ist, daß der Impfstoff als Immuntherapeutikum zum Einsatz nach der Infektion eingesetzt wird und ebenfalls das Variantenspektrum dieses Subtyps abdeckt. Die Vakzine wird entweder zur Injektion mit Adjuvants verabreicht oder als orale, genitale oder rectale Applikationsform z.B. über Nanopartikel.

Weiterhin ist auch ein Verfahren zur Herstellung einer Vakzine gegen HIV-1-Virusinfektionen aus den Viren HIV-1_{D757}, HIV-1_{D747} und HIV-1_{D760} sowie die Verwendung dieser Viren zur Herstellung einer Vakzine Gegenstand der Erfindung.

In einer weiteren Ausführungsform werden die Viren HIV-1_{D757}, HIV-1_{D747} und HIV-1_{D760} in ein geeignetes Wirtstier übertragen und dort vermehrt. Die gebildeten Antikörper werden sodann nach entsprechender Aufarbeitung, wie dies auch im Stand der Technik für andere Impfstoffe bekannt ist, als Passiv-Impfstoff gegen HIV-Virusinfektionen verwendet. Als Wirtstiere können insbesondere geeignete Affenarten verwendet werden, aber auch Tiere, die immunisiert werden können ohne HIV-Symptome zu entwickeln (z.B. Kaninchen) (Filice et al., Nature 335, 1988, p. 366 bis 369).

Auch humane monoklonale Antikörper gegen HIV-1_{D757}, HIV-1_{D747} und HIV-1_{D760} sind zur Herstellung einer Vakzine zum Schutz vor HIV-1-Virusinfektionen dieses Subtyps geeignet.

In einer weiteren Ausführungsform wird in einem geeigneten Vektor DNA, von der eine zu der viralen m-RNA komplementären RNA vollständig oder partiell transkribiert werden kann, in menschliche Zellen des Immunsystems transfiziert und so in den Menschen gebracht. Die Virale m-RNA wird somit kompetitiv vom weiteren Vermehrungszyklus ausgeschlossen. Desgleichen können synthetische Oligonukleotide zur Neutralisierung der viralen m-RNA angewendet werden.

Neben den Anwendungen zur Therapie oder zur Impfstoffherstellung können HIV-1_{D757}, HIV-1_{D747} und HIV-1_{D760} aber auch zur Differentialdiagnostik eingesetzt werden, um Infektionen mit diesem Subtyp von anderen Subtypen zu unterscheiden. Für die Differentialdiagnostik werden ausgewählte Bereiche der DNA von HIV-1_{D757}, HIV-1_{D747} und HIV-1_{D760} herangezogen, die bei dem Prototyp HIV-1_{Lai} entweder gar nicht vorkommen oder erheblich abweichen (mehr als 30 %). Von diesen Bereichen werden Peptide oder Nukleinsäuren für die Diagnostik nach den üblichen Methoden hergestellt (Markierung mit radioaktiven Isotopen, Immunfluoreszenztest, ELISA, etc.).

Es ist deshalb ebenso Bestandteil dieser Erfindung, HIV-1_{D757}, HIV-1_{D747} und HIV-1_{D760} oder Antigene, Peptide oder Nukleinsäuren davon, für die differentielle Diagnostik zur Unterscheidung zwischen Infektionen des Subtyps, charakterisiert durch HIV-1_{D757}, HIV-1_{D747} oder HIV-1_{D760}, und solchen der anderen Subtypen von HIV-1, definiert durch die Prototypen HIV-1_{Lai} (USA/Europa), HIV-1_{Mal} (Zentralafrika), HIV-1_{U455} (Uganda/Rwanda) und Nordthailand einzusetzen.

Es ist auch mögliche, Peptidreste oder PCR-Tests auf der Basis des HIV-1_{D757}, HIV-1_{D747} oder HIV-1_{D760} einzusetzen, mit deren Hilfe man Viren dieses Subtyps von anderen HIV-1 Viren unterscheiden kann.

Im folgenden werden die Figuren sowie die Tabelle beschrieben:

Die Tabelle zeigt die Nukleotid- und Aminosäuresequenzhomologie zwischen HIV-1_{D757}, HIV-1_{D747} oder HIV-1_{D760} und anderen Subtypen der HIV-1 Viren in Prozent. Die Sequenzen wurden verglichen unter Benutzung von Mikrogenie ™-Sequenz Software der Fa. Beckmann.
Figur 1 zeigt die Aminosäuresequenzen in Einzelbuchstabenschreibweise der drei indischen env-Klone im Vergleich zu den entsprechenden Abschnitten von HIV-1 Viren anderer Subtypen. Hervorgehoben sind die beiden Peptide 1 und 2, in denen sich der indische Subtyp hauptsächlich von den anderen Sequenzen unterscheidet sowie die zusätzliche Glycosylierungsstelle (unterstrichen) in unmittelbarer Nachbarschaft zur CD4-Bindungsdomäne.
   (-) symbolisiert identische Aminosäuren
   (.) symbolisiert fehlende Aminosäuren
Figur 2 stellt die Beziehung der Sequenzen HIV-1_{D757}, HIV-1_{D747} oder HIV-1_{D760} zu den übrigen Viren im phylogenetischen Stammbau der Viren dar. Dieser Stammbar wurde unter Benutzung von PAUP (Smith, T.F. et al., Nature 333, 573 bis 575, 1988) und der Version 3.21 des PHYLIP bootstrappin algorythmus erstellt.

**Tabelle**

| Homologie von indischen HIV-1 Isolaten und amerikanisch/europäischen bzw. prototypischen afrikanischen HIV-1 Sequenzen (1,8 kb PCR-Fragment korrespondierend zu Positionen 6129 -7976 von HIV-1Lai) | | | | | |
|---|---|---|---|---|---|
| HIV-1 | Lai | SF2 | Eli | Mal | SIVepz |
| D757 | 79.7 (75.7) | 81.6 (75.9) | 81.2 (74.2) | 80.6 (73.7) | 66.1 (61.2) |
| D747 | 79.4 (73.5) | 80.9 (75.6) | 80.7 (72.8) | 78.9 (73.2) | 65.8 (63.2) |
| D760 | 80.7 (72.1) | 81.4 (73.6) | 80.9 (71.2) | 80.2 (71.7) | 66.1 (61.0) |
| Lai | | 90.0 (84.1) | 83.7 (76.2) | 82.8 (74.3) | 65.0 (60.1) |
| SF2 | | | 83.8 (75.6) | 82.8 (74.6) | 64.3 (62.0) |
| Eli | | | | 85.2 (76.9) | 65.7 (61.9) |
| Mal | | | | | 66.4 (63.2) |

Figur 1

HIV-1Bru -----------S-K--DLGNATN-NSSN.TNSSSGEMMMEKG---------IS-
HIV-1SF2 -----------------DLGKATN-NSSN.WKEEI......KG--------I--
HIV-1D757 SLWDQSLKPCVKLTPLCVTLNCTNA...NVTYDN.........GNYTEEIKNCSFNTTT
HIV-1D747 -----------H----TYS-S--NS..........T.-N-----------
HIV-1D760 -----------E-G-VNAT-I-NNGE...NNPTNIT--R------P--A--
HIV-1Eli -----------SD-...ELRNNGTMGNNVTTEEK....GM-------V--
HIV-1Mal -----------VNGTA-NGT-AGSNRTNAELKMEIG-V-------I-P
SIVcpz -----------Q-SK-NFSQA.........KNLTNQTSSPPL-M-------V--

HIV-1Bru SI-GKV--EY-F----II-I-NDTT-.....-T-TS----V-------E---
HIV-1SF2 SI--KI--EN---RN-----I-NASTTTNYTN-----H--R-V-------E---
HIV-1D757 ELRDQKQKVAALFYKLDVVPLDGNDNSS....YRLINCNTSAITQACPKVSFDPIPIHYC
HIV-1D747 ----K----Q-------NTT-.....-----------------------
HIV-1D760 -I--RQ----Y----R--I---N-N-T...-----------T-------
HIV-1Eli V-K-K--Q-Y----R--I--I-NDSSTNS.TN-----------E-----
HIV-1Mal VGS-KR-E.Y-T-N--L-QI-D...-DN.SS-------V-------T-----
SIVcpz ---K-KQ-YS---VE---N-GNEN-T....-I-----T-----T--E---

----F----------------T----VQ-----R---------------E-VV---A-FT
T---F-----------K----T----VQ-----R-I-------------E-VV---D-FT
APAGYAILKCNNKTFNGTGPCHNVSTRTCTHGIKPVVSTQLLLNGSLAEGEIIIRSENLA
-G---------------------I--VQ-------------------------------T
--------------------------VQ-----S-------------------------T
----F-----RD-K-------T----VQ-----R---------------E-V-------T
----F------D-K------EI-K--VQ---------------------E-M-------T
----F------D-D-S-K-K-T----VH-----------T-------I--N-TV-V--KS

D-A-----Q-----E-N--------------QR---RA-VTI-K-.NM-----RA-
--A-----Q---E--A-N-----------Y-..---RA-HT--R-----K-----RAQ
NNVKTIIVHLNQSVRIVCTRPNNNTRKSIRI..GPGQTFYATGDIIGDIRQAHCNISEGK
-----------E--Y-----------GV-..-----Q-----------KH-
D----------EV-------------..-----Q-----------D-
--A-N--A---E--K-T-A--YQ--QRTP-..--L--SL-T-R.SRSI-G-----RAQ
D-T-N--Q--ET-T-N---G---RG-HF-..---AL-T--.-V----R-Y-T-N-TE
K-TDVW--Q-VEA-SLN-H--G---GEVQ-..---M---NIENVV--T-S-Y-K-NGTT

HIV-1_{Bru} --A--KQIAS--R-Q-G-N--.-I-KQ----DP--V-------G-------STQ-----WF
HIV-1_{SF2} --N--EQIV---R-Q-G-N--.-V-NQ----DP--VM---------------TQLF....
HIV-1_{D757} WNETLQRVGKKLAEYFPN.KT.IKFASSSGGGLEITTHSFNCRGEFFYCNTSNLFNSTY.
HIV-1_{D747} -----------H---.---R-------D-------------D---G---.
HIV-1_{D760} -----------H---.-----A----D-------Y-------G---G---.
HIV-1_{Eli} -SK---Q-AR--GTLL.-KTI.---KP-----DP-------G-------G-----WN
HIV-1_{Mal} -DK---Q-AV--GSLL.-KTK.-I-N-----DP-------K-----WQ
SIVcpz --R-VEE-K-A--TSSNRTAAN-TLNRA---DP-V-H-M---G-------QI-....

HIV-1Bru NSTWSTEGSNNTEGSD---LP----F--------K--------S-Q-R-S------T-
HIV-1SF2 NNTWRLNHTEGTKG-D--ILP-----------K--------G-Q-S-S------T-
HIV-1D757 .....MPNGTKGNSNSTITIQCRIKQIINMWQEVGRAMYAPPIEGNITCESNITGLLLVR
HIV-1D747 ......------S--S----P-----------------A-----K------I---
HIV-1D760 ......------S-L-----P------V-L-------FAR----K---------
HIV-1Eli ISAWNNITESNNST-TN--L------K-.VAGRK-I------R--L-S------T-
HIV-1Mal NNG.ARLSNSTESTG-.--LP-------KT-K-------A-V-N-L------I-T-
SIVcpz ........-DNIT-GI-ILP--R--VSS-MR---GI-------R-----N-------TS

HIV-1Bru ---NN.--G.S-I--------D---------K-E-------K-------.....Q---
HIV-1SF2 ---NVT-D.T-V---------D---------IK-E---I---K-------.....Q---
HIV-1D757 DGGTESNNT..ETFRPGGG-MRNNWRSELYKYKVVEIKPLGVAPTTAKRRVV.....EREK
HIV-1D747 ---IEL-D-KT------E--D-----------------------------.....----
HIV-1D760 ---EDT-D..T-I-S------D-----------------------------.....----
HIV-1Eli ---..I--STN---------D---------Q-E-------R-------.....----
HIV-1Mal ---NS-D-SDN--L-------D--I-------R-E-------K-------.....----
SIVcpz -TPVTN-SGNL.----T-N-KDI---------R-E--S---K-R-HT-ARQKD-Q-

HIV-1Bru -----.---L------------------R----------N--------------------
HIV-1SF2 -----V--M-------------------V-L--------N--------------------
HIV-1D757 RAVGI.GAVFLGFLGAAGSTMGAASMTLTVQARQLLSGIVQQQSNLLRAIEAQQHLLQLT
HIV-1D747 -----.-----------------------V------------------------------
HIV-1D760 ----L.----------------------I-------------------------------
HIV-1Eli --I-L.---M------------------R-V-----M--N--------------------
HIV-1Mal --I-L.---M--------------------L--------N--------------------
SIVcpz --AFGL--L-------------------AV---------N---K---------------S

HIV-1Bru -----------A-I-V------------C------A---A------.-K-LEQ-------
HIV-1SF2 -----------A---V----R-------C------A---A------.-K-LED-------
HIV-1D757 VWGIKQLQTRVLAIERYLKDQQLLGIWGRSGKLICTTNVPWNSSWS.NRSQTDIWDNMTW
HIV-1D747 I----------A--------E-------C------T-----------.-------------
HIV-1D760 -------------------------M--C------A----------.-------------
HIV-1Eli -----------A-I-V------------C------H----------.---LNE--Q----
HIV-1Mal -----------A---V----Q--R-M--C------H---F------.---LD--N-----
SIVcpz I--V-------A-L-V----Q--I-L--C------AV-Y-T----PGSN-TD---G-L--

HIV-1Bru -E----.......................................--N---SL-HS-I-E

HIV-1SF2 ---E-........................................--D---N---T---E

HIV-1D757 MQWDR........................................EISNYTDTIYRLLED

HIV-1D747 ------.......................................------E--------

HIV-1D760 ------.......................................------N--------

HIV-1Eli -E-E-........................................--D---GL--S-I-E

HIV-1Mal ---EK........................................------GI--N-I-E

SIVcpz Q---KLVSNYTGKIFGLLEEAQSQQEKNERDLLELDQWASLWNWFD-T---GK-FG---E

HIV-1Bru ------K--QE--E--K-AS-----N------------------V--R-V----------
HIV-1SF2 ------K--QE--E--K-AS------------------------V--R-V----------
HIV-1D757 SQNQQERNEKDLLALDSWKNLWNWFSITNWLWYIKIFIMIVGGLIGLKIIFAVLSIVNRV
HIV-1D747 --------------------------------------I--------R------C-----
HIV-1D760 -----------------------------------------------R------------
HIV-1Eli --T---K---E--E--K-AS--------Q-----------I------R-V----L-----
HIV-1Mal --I---K---E--E--K-AS-------SK---R-----IV-------R------L-----
SIVcpz A-S---K---R--E--Q-AS-----D--K---------L-A--I---R---MT-F-V-R-

HIV-1Bru -----------
HIV-1SF2 -----------
HIV-1D757 RQGYSPLSFQT
HIV-1D747 KA---------
HIV-1D760 -----------
HIV-1Eli -----------
HIV-1Mal --------L--
SIVcpz --------L--

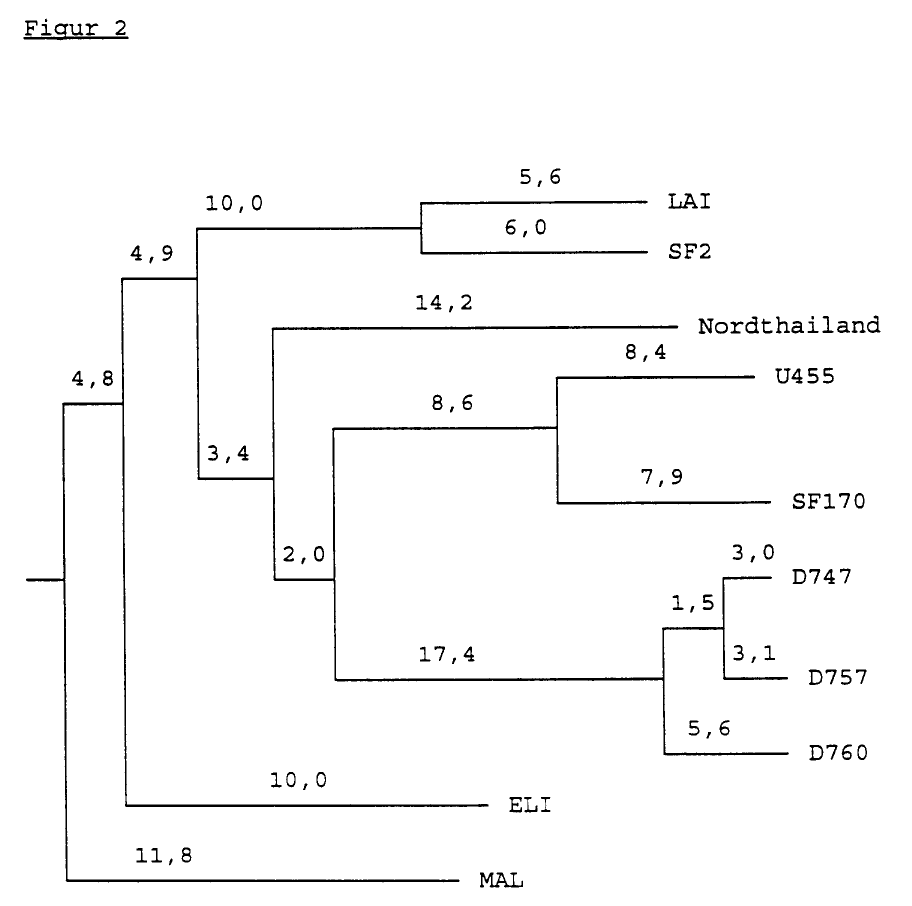

## Patentansprüche

1. Virusisolat HIV-1_{D747} (ECACC V92082718)
Virusisolat HIV-1_{D757} (ECACC V92082719) und
Virusisolat HIV-1_{D760} (ECACC V92082720).

2. Vakzine gegen HIV-1-Virusinfektionen des durch die Virusisolate gemäß Anspruch 1 definierten indischen Subtyps, dadurch gekennzeichnet, daß sie aus dem Virus HIV-1_{D757}, dem Virus HIV-1_{D747} und/ oder dem Virus HIV-1_{D760} hergestellt ist.

3. Vakzine nach Anspruch 2 enthaltend als Wirkstoff die Peptide
MPNGTKSNS, MPNGTKGNS, MPNGTKSNL,
RNEKDLLALDSWKN,
Peptide aus der CD4-Bindungsregion mit einer zusätzlichen Glycosylierungsstelle unmittelbar vor dem Cystein 450 (bezogen auf den Prototyp HIV-1_{Lai}) oder
Kombinationen mit diesen Peptiden.

4. Vakzine gemäß Anspruch 2, dadurch gekennzeichnet, daß die Peptide durch chemische Synthese hergestellt und gegebenenfalls chemisch modifiziert sind.

5. Vakzine gegen HIV-1-Virusinfektionen nach Anspruch 2 enthaltend als Wirkstoff Peptide aus variablen Bereichen von HIV-1_{D757}, HIV-1_{D747} und/oder HIV-1_{D760}, die bei den anderen HIV-1 Subtypen nicht vorkommen.

6. Vakzine gemäß der Ansprüche 2 bis 5, dadurch gekennzeichnet, daß sie als Immuntherapeutikum nach der Infektion eingesetzt wird.

7. Verfahren zur Herstellung einer Vakzine nach den Ansprüchen 2 bis 5, dadurch gekennzeichnet, daß die Viren HIV-1_{D757}, HIV-1_{D747} und/oder HIV-1_{D760} eingesetzt werden.

8. Verfahren zur Herstellung eines Passiv-Impfstoffs gegen HIV-Virusinfektionen des durch die Virusisolate gemäß Anspruch 1 definierten indischen Subtyps, dadurch gekennzeichnet, daß er aus Antikörpern gegen die Viren HIV-1_{D757}, HIV-1_{D747} und/oder HIV-1_{D760} hergestellt ist.

9. Verwendung der Virusisolate HIV-1_{D757}, HIV-1_{D747} und/ oder HIV-1_{D760} gemäß Anspruch 1 zur Herstellung von Vakzine zum Schutz vor HIV-1-Virusinfektionen und zur Herstellung von Vakzinen zum therapeutischen Einsatz nach der HIV-1-Virusinfektion.

10. Verwendung der Virusisolate nach Anspruch 1 für die Diagnostik zur Differenzierung von HIV-1-Virus-Subtypen.

## Claims

1. The virus isolate HIV-1_{D747} (ECACC V92082718),
the virus isolate HIV-1_{D757} (ECACC V92082719), and
the virus isolate HIV-1_{D760} (ECACC V92082720).

2. A vaccine against HIV-1 virus infections of the Indian subtype defined by the virus isolates according to claim 1, characterized by being prepared from the virus HIV-1_{D757}, the virus HIV-1_{D747} and/or the virus HIV-1_{D760}.

3. A vaccine according to claim 2, containing as the active ingredient the peptides
MPNGTKSNS, MPNGTKGNS, MPNGTKSNL,
RNEKDLLALDSWKN,
peptides from the CD4-binding region comprising an additional glycosylation site immediately in front of cysteine 450 (relative to the prototype HIV-1_{Lai}), or
combinations of said peptides.

4. The vaccine according to claim 2, characterized in that the peptides are produced by chemical synthesis and are optionally chemically modified.

5. The vaccine against HIV-1 virus infections according to claim 2, containing as the active ingredient peptides from variable regions of HIV-1_{D757}, HIV-1_{D747} and/or HIV-1_{D760} which do not occur in the other HIV-1 subtypes.

6. The vaccine according to claims 2 to 5, characterized by being used as an immunotherapeutic agent after infection.

7. A process for producing a vaccine according to claims 2 to 5, characterized in that the viruses HIV-1_{D757}, HIV-1_{D747} and/or HIV-1_{D760} are employed.

8. A process for producing a passive vaccine against HIV-1 virus infections of the Indian subtype defined by the virus isolates according to claim 1, characterized by being prepared from antibodies against the viruses HIV-1_{D757}, HIV-1_{D747} and/or HIV-1_{D760}.

9. Use of the virus isolates HIV-1_{D757}, HIV-1_{D747} and/or HIV-1_{D760} according to claim 1 for producing a vaccine for protection against HIV-1 virus infections and for producing vaccines for therapeutic use after HIV-1 infection.

10. Use of the virus isolates according to claim 1 for differential diagnostics between HIV-1 viruses of different subtypes.

## Revendications

1. Isolat de virus VIH-lD747 (ECACC V92082718),
Isolat de virus VIH-lD757 (ECACC V92082719), et
Isolat de virus VIH-lD760 (ECACC V92082720).

2. Vaccin contre les infections par le virus VIH-l du sous-type indien défini par les isolats de virus selon la revendication 1, caractérisé en ce qu'il est préparé à partir du virus VIH-lD757, du virus VIH-lD747 et/ou du virus VIH-lD760.

3. Vaccin selon la revendication 2, contenant en tant que principe actif les peptides MPNGTKSNS, MPNGTKGNS, MPNGTKSNL, RNEKDLLALDSWKN, des peptides de la région de fixation du CD4, avec un site supplémentaire de glycosylation immédiatement en amont de la cystéine 450 (par rapport au prototype VIH-lLai), ou des combinaison de ces peptides.

4. Vaccin selon la revendication 2, caractérisé en ce que les peptides sont préparés par synthèse chimique et éventuellement sont chimiquement modifiés.

5. Vaccin contre les infections par le virus VIH-l selon la revendication 2, contenant en tant que principe actif des peptides de la région variable du VIH-lD757, du VIH-lD747 et/ou du VIH-lD760, qui n'apparaissent pas dans les autres sous-types du VIH-l.

6. Vaccin selon les revendications 2 à 5, caractérisé en ce qu'il est utilisé en immunothérapie après l'infection.

7. Procédé de préparation d'un vaccin selon les revendications 2 à 5, caractérisé en ce qu'on utilise les virus VIH-lD757, VIH-lD747 et/ou VIH-lD760.

8. Procédé de préparation d'un vaccin passif contre des infections par le virus VIH du sous-type indien défini par les isolats de virus selon la revendication 1, caractérisé en ce qu'il est préparé à partir d'anticorps contre les virus VIH-lD757, VIH-lD747 et/ou VIH-lD760.

9. Utilisation des isolats de virus VIH-lD757, VIH-lD747 et/ou VIH-lD760 selon la revendication 1 pour préparer des vaccins assurant une protection contre les infections par le virus VIH-l, et pour préparer des vaccins destinés à une utilisation thérapeutique après infection par le virus VIH-l.

10. Utilisation des isolats de virus selon la revendication 1 pour le diagnostic servant à la différenciation de sous-types de virus VIH-l.


SEQUENZPROTOKOLL

(1) ALLGEMEINE INFORMATION:

(i) ANMELDER:
(A) NAME: Chemotherapeutisches Forschungsinstitut
Georg-Speyer-Haus
(B) STRASSE: Paul-Ehrlich-Strasse 42-44
(C) ORT: Frankfurt am Main
(E) LAND: Deutschland
(F) POSTLEITZAHL: 60596

(ii) ANMELDETITEL: Neue HIV-1-Virusisolate eines Subtyps,
Vakzine gegen HIV-1-Virusinfektionen dieses Subtyps und
Verfahren zu ihrer Herstellung, Verwendung der
HIV-1-Virusisolate

(iii) ANZAHL DER SEQUENZEN: 10

(iv) COMPUTER-LESBARE FORM:
(A) DATENTRÄGER: Floppy disk
(B) COMPUTER: IBM PC compatible
(C) BETRIEBSSYSTEM: PC-DOS/MS-DOS
(D) SOFTWARE: PatentIn Release #1.0, Version #1.25 (EPA)

(2) INFORMATION ZU SEQ ID NO: 1:

(i) SEQUENZ CHARAKTERISTIKA:
(A) LÄNGE: 1932 Basenpaare
(B) ART: Nukleinsäure
(C) STRANGFORM: nicht bekannt
(D) TOPOLOGIE: nicht bekannt

(ii) ART DES MOLEKÜLS: DNS (genomisch)

(vi) URSPRÜNLICHE HERKUNFT:
(A) ORGANISMUS: HIV-1D757

(ix) MERKMALE:
(A) NAME/SCHLÜSSEL: CDS
(B) LAGE: 2..1789

(xi) SEQUENZBESCHREIBUNG: SEQ ID NO: 1:

C AGT TTA TGG GAT CAA AGC CTA AAG CCA TGT GTA AAG TTG ACC CCA 46
Ser Leu Trp Asp Gln Ser Leu Lys Pro Cys Val Lys Leu Thr Pro
1               5                   10                  15

CTC TGT GTC ACT TTA AAT TGT ACA AAT GCA AAT GTT ACC TAT GAT AAT 94
Leu Cys Val Thr Leu Asn Cys Thr Asn Ala Asn Val Thr Tyr Asp Asn
                20                  25                  30

GGT AAC TAC ACT GAA GAA ATA AAA AAT TGC TCT TTC AAT ACA ACT ACA 142
Gly Asn Tyr Thr Glu Glu Ile Lys Asn Cys Ser Phe Asn Thr Thr Thr
            35                  40                  45

GAA CTA AGA GAT CAG AAA CAG AAA GTT GCT GCA CTT TTT TAT AAA CTT 190
Glu Leu Arg Asp Gln Lys Gln Lys Val Ala Ala Leu Phe Tyr Lys Leu
        50                  55                  60

GAT GTA GTA CCA CTT GAT GGT AAT GAT AAC TCT AGT TAT AGA TTA ATA 238
Asp Val Val Pro Leu Asp Gly Asn Asp Asn Ser Ser Tyr Arg Leu Ile
    65                  70                  75

AAT TGT AAT ACC TCA GCC ATA ACA CAA GCC TGT CCA AAG GTC TCT TTT 286
Asn Cys Asn Thr Ser Ala Ile Thr Gln Ala Cys Pro Lys Val Ser Phe
80                  85                  90                  95

GAC CCA ATC CCT ATA CAT TAT TGT GCT CCA GCT GGT TAT GCG ATT CTA 334
Asp Pro Ile Pro Ile His Tyr Cys Ala Pro Ala Gly Tyr Ala Ile Leu
                100                 105                 110

AAG TGT AAT AAT AAG ACA TTC AAT GGG ACA GGA CCA TGC CAT AAT GTC 382
Lys Cys Asn Asn Lys Thr Phe Asn Gly Thr Gly Pro Cys His Asn Val
            115                 120                 125

AGC ACA CGT ACA TGT ACA CAT GGA ATT AAG CCA GTA GTA TCA ACT CAA 430
Ser Thr Arg Thr Cys Thr His Gly Ile Lys Pro Val Val Ser Thr Gln
        130                 135                 140

CTA CTG TTA AAT GGT AGC CTA GCA GAA GGA GAG ATA ATA ATT AGA TCT 478
Leu Leu Leu Asn Gly Ser Leu Ala Glu Gly Glu Ile Ile Ile Arg Ser
    145                 150                 155

GAA AAT CTG GCA AAC AAT GTC AAA ACA ATA ATA GTA CAT CTT AAT CAA 526
Glu Asn Leu Ala Asn Asn Val Lys Thr Ile Ile Val His Leu Asn Gln
160                 165                 170                 175

TCT GTA AGA ATT GTG TGT ACA AGA CCC AAC AAT AAT ACA AGA AAA AGT 574
Ser Val Arg Ile Val Cys Thr Arg Pro Asn Asn Asn Thr Arg Lys Ser
                180                 185                 190

ATA AGG ATA GGA CCA GGA CAA ACA TTC TAT GCA ACA GGA GAC ATA ATA 622
Ile Arg Ile Gly Pro Gly Gln Thr Phe Tyr Ala Thr Gly Asp Ile Ile
            195                 200                 205

GGA GAC ATA AGA CAA GCA CAT TGT AAC ATT AGT GAA GGT AAA TGG AAT 670
Gly Asp Ile Arg Gln Ala His Cys Asn Ile Ser Glu Gly Lys Trp Asn
        210                 215                 220

GAA ACT TTA CAA AGG GTA GGT AAA AAA TTA GCA GAA TAC TTC CCT AAT 718
Glu Thr Leu Gln Arg Val Gly Lys Lys Leu Ala Glu Tyr Phe Pro Asn
    225                 230                 235

AAA ACA ATA AAA TTT GCA TCA TCC TCA GGA GGG GGC CTA GAA ATT ACA 766
Lys Thr Ile Lys Phe Ala Ser Ser Ser Gly Gly Gly Leu Glu Ile Thr
240                 245                 250                 255

ACA CAT AGC TTT AAT TGT AGA GGA GAA TTT TTC TAT TGC AAT ACA TCA 814
Thr His Ser Phe Asn Cys Arg Gly Glu Phe Phe Tyr Cys Asn Thr Ser
                260                 265                 270

AAC CTG TTT AAT AGT ACA TAC ATG CCT AAT GGT ACA AAA GGT AAT TCA 862
Asn Leu Phe Asn Ser Thr Tyr Met Pro Asn Gly Thr Lys Gly Asn Ser
            275                 280                 285

AAC TCA ACC ATC ACA ATC CAA TGC AGA ATA AAA CAA ATT ATA AAC ATG 910
Asn Ser Thr Ile Thr Ile Gln Cys Arg Ile Lys Gln Ile Ile Asn Met
        290                 295                 300

TGG CAG GAG GTA GGA CGA GCA ATG TAT GCC CCT CCC ATT GAA GGA AAC 958
Trp Gln Glu Val Gly Arg Ala Met Tyr Ala Pro Pro Ile Glu Gly Asn
    305                 310                 315

ATA ACG TGT GAA TCC AAT ATC ACA GGA CTA CTA TTG GTA CGT GAT GGA 1006
Ile Thr Cys Glu Ser Asn Ile Thr Gly Leu Leu Leu Val Arg Asp Gly
320                 325                 330                 335

GGA ACA GAG TCA AAT AAT ACA GAG ACA TTC AGA CCT GGA GGA GGA GAT 1054
Gly Thr Glu Ser Asn Asn Thr Glu Thr Phe Arg Pro Gly Gly Gly Asp
                340                 345                 350

ATG AGG AAC AAT TGG AGA AGT GAA TTA TAT AAA TAT AAA GTG GTA GAA 1102
Met Arg Asn Asn Trp Arg Ser Glu Leu Tyr Lys Tyr Lys Val Val Glu
            355                 360                 365

ATT AAG CCA TTG GGA GTA GCG CCC ACT ACT GCA AAA AGG AGA GTG GTG 1150
Ile Lys Pro Leu Gly Val Ala Pro Thr Thr Ala Lys Arg Arg Val Val
        370                 375                 380

GAG AGA GAA AAA AGA GCA GTG GGA ATA GGA GCT GTG TTC CTT GGG TTC 1198
Glu Arg Glu Lys Arg Ala Val Gly Ile Gly Ala Val Phe Leu Gly Phe
    385                 390                 395

TTG GGA GCA GCA GGA AGC ACT ATG GGC GCG GCA TCA ATG ACG CTG ACG 1246
Leu Gly Ala Ala Gly Ser Thr Met Gly Ala Ala Ser Met Thr Leu Thr
400                 405                 410                 415

GTA CAG GCC AGA CAA TTG TTG TCT GGT ATA GTG CAA CAG CAA AGC AAT 1294
Val Gln Ala Arg Gln Leu Leu Ser Gly Ile Val Gln Gln Gln Ser Asn
                420                 425                 430

TTG CTG AGG GCT ATA GAG GCG CAA CAG CAT CTG TTG CAA CTC ACG GTC 1342
Leu Leu Arg Ala Ile Glu Ala Gln Gln His Leu Leu Gln Leu Thr Val
            435                 440                 445

TGG GGC ATT AAG CAG CTC CAG ACA AGA GTC CTG GCT ATA GAA AGA TAC 1390
Trp Gly Ile Lys Gln Leu Gln Thr Arg Val Leu Ala Ile Glu Arg Tyr
        450                 455                 460

CTA AAG GAT CAA CAG CTC CTA GGG ATT TGG GGC CGC TCT GGA AAA CTC 1438
Leu Lys Asp Gln Gln Leu Leu Gly Ile Trp Gly Arg Ser Gly Lys Leu
    465                 470                 475

ATC TGC ACC ACT AAT GTA CCT TGG AAC TCC AGC TGG AGT AAC AGA TCT 1486
Ile Cys Thr Thr Asn Val Pro Trp Asn Ser Ser Trp Ser Asn Arg Ser
480                 485                 490                 495

CAA ACA GAT ATT TGG GAT AAC ATG ACC TGG ATG CAG TGG GAT AGA GAA 1534
Gln Thr Asp Ile Trp Asp Asn Met Thr Trp Met Gln Trp Asp Arg Glu
                500                 505                 510

ATT AGT AAT TAC ACA GAC ACA ATA TAC AGG TTG CTT GAA GAC TCG CAA 1582
Ile Ser Asn Tyr Thr Asp Thr Ile Tyr Arg Leu Leu Glu Asp Ser Gln
            515                 520                 525

AAC CAG CAG GAA AGA AAT GAA AAA GAT TTA TTA GCA TTG GAC AGT TGG 1630
Asn Gln Gln Glu Arg Asn Glu Lys Asp Leu Leu Ala Leu Asp Ser Trp
        530                 535                 540

AAA AAT CTG TGG AAT TGG TTT AGC ATA ACA AAT TGG CTG TGG TAT ATA 1678
Lys Asn Leu Trp Asn Trp Phe Ser Ile Thr Asn Trp Leu Trp Tyr Ile
    545                 550                 555

AAA ATA TTC ATA ATG ATA GTA GGA GGC TTG ATA GGT TTG AAA ATA ATT 1726
Lys Ile Phe Ile Met Ile Val Gly Gly Leu Ile Gly Leu Lys Ile Ile
560                 565                 570                 575

TTT GCT GTG CTC TCT ATA GTG AAT AGA GTT AGG CAG GGA TAC TCA CCT     1774
Phe Ala Val Leu Ser Ile Val Asn Arg Val Arg Gln Gly Tyr Ser Pro
                580                 585                 590

TTA TCG TTT CAG ACC CTTACCCCGA ACCCAGGGGG ACCCGACAGG CTCGAAAGAA     1829
Leu Ser Phe Gln Thr
            595

TCGAAGGAGG AGGTGGAGAG CAAGACAAAG ACAGATCCAT TCGCTTAGTG AACGGATTCT     1889

TAGCACTTGC CTGGGACGAC TGCGGAGCCT GTGCCTCTTC AGC     1932

(2) INFORMATION ZU SEQ ID NO: 2:

(i) SEQUENZ CHARAKTERISTIKA:
(A) LÄNGE: 596 Aminosäuren
(B) ART: Aminosäure
(D) TOPOLOGIE: linear

(ii) ART DES MOLEKÜLS: Protein

(xi) SEQUENZBESCHREIBUNG: SEQ ID NO: 2:

Ser Leu Trp Asp Gln Ser Leu Lys Pro Cys Val Lys Leu Thr Pro Leu
1               5                   10                  15

Cys Val Thr Leu Asn Cys Thr Asn Ala Asn Val Thr Tyr Asp Asn Gly
            20                  25                  30

Asn Tyr Thr Glu Glu Ile Lys Asn Cys Ser Phe Asn Thr Thr Thr Glu
        35                  40                  45

Leu Arg Asp Gln Lys Gln Lys Val Ala Ala Leu Phe Tyr Lys Leu Asp
    50                  55                  60

Val Val Pro Leu Asp Gly Asn Asp Asn Ser Ser Tyr Arg Leu Ile Asn
65                  70                  75                  80

Cys Asn Thr Ser Ala Ile Thr Gln Ala Cys Pro Lys Val Ser Phe Asp
                85                  90                  95

Pro Ile Pro Ile His Tyr Cys Ala Pro Ala Gly Tyr Ala Ile Leu Lys
            100                 105                 110

Cys Asn Asn Lys Thr Phe Asn Gly Thr Gly Pro Cys His Asn Val Ser
        115                 120                 125

Thr Arg Thr Cys Thr His Gly Ile Lys Pro Val Val Ser Thr Gln Leu
    130                 135                 140

Leu Leu Asn Gly Ser Leu Ala Glu Gly Glu Ile Ile Ile Arg Ser Glu
145                 150                 155                 160

Asn Leu Ala Asn Asn Val Lys Thr Ile Ile Val His Leu Asn Gln Ser
                165                 170                 175

Val Arg Ile Val Cys Thr Arg Pro Asn Asn Asn Thr Arg Lys Ser Ile
            180                 185                 190

Arg Ile Gly Pro Gly Gln Thr Phe Tyr Ala Thr Gly Asp Ile Ile Gly
        195                 200                 205

Asp Ile Arg Gln Ala His Cys Asn Ile Ser Glu Gly Lys Trp Asn Glu
    210                 215                 220

Thr Leu Gln Arg Val Gly Lys Lys Leu Ala Glu Tyr Phe Pro Asn Lys
225                 230                 235                 240

Thr Ile Lys Phe Ala Ser Ser Ser Gly Gly Gly Leu Glu Ile Thr Thr
                245                 250                 255

His Ser Phe Asn Cys Arg Gly Glu Phe Phe Tyr Cys Asn Thr Ser Asn
            260                 265                 270

Leu Phe Asn Ser Thr Tyr Met Pro Asn Gly Thr Lys Gly Asn Ser Asn
        275                 280                 285

Ser Thr Ile Thr Ile Gln Cys Arg Ile Lys Gln Ile Ile Asn Met Trp
    290                 295                 300

Gln Glu Val Gly Arg Ala Met Tyr Ala Pro Pro Ile Glu Gly Asn Ile
305                 310                 315                 320

Thr Cys Glu Ser Asn Ile Thr Gly Leu Leu Leu Val Arg Asp Gly Gly
                325                 330                 335

Thr Glu Ser Asn Asn Thr Glu Thr Phe Arg Pro Gly Gly Gly Asp Met
            340                 345                 350

Arg Asn Asn Trp Arg Ser Glu Leu Tyr Lys Tyr Lys Val Val Glu Ile
        355                 360                 365

Lys Pro Leu Gly Val Ala Pro Thr Thr Ala Lys Arg Arg Val Val Glu
    370                 375                 380

Arg Glu Lys Arg Ala Val Gly Ile Gly Ala Val Phe Leu Gly Phe Leu
385                 390                 395                 400

Gly Ala Ala Gly Ser Thr Met Gly Ala Ala Ser Met Thr Leu Thr Val
                405                 410                 415

Gln Ala Arg Gln Leu Leu Ser Gly Ile Val Gln Gln Gln Ser Asn Leu
            420                 425                 430

Leu Arg Ala Ile Glu Ala Gln Gln His Leu Leu Gln Leu Thr Val Trp
        435                 440                 445

Gly Ile Lys Gln Leu Gln Thr Arg Val Leu Ala Ile Glu Arg Tyr Leu
    450                 455                 460

Lys Asp Gln Gln Leu Leu Gly Ile Trp Gly Arg Ser Gly Lys Leu Ile
465                 470                 475                 480

Cys Thr Thr Asn Val Pro Trp Asn Ser Ser Trp Ser Asn Arg Ser Gln
                485                 490                 495

Thr Asp Ile Trp Asp Asn Met Thr Trp Met Gln Trp Asp Arg Glu Ile
            500                 505                 510

Ser Asn Tyr Thr Asp Thr Ile Tyr Arg Leu Leu Glu Asp Ser Gln Asn
        515                 520                 525

Gln Gln Glu Arg Asn Glu Lys Asp Leu Leu Ala Leu Asp Ser Trp Lys
    530                 535                 540

Asn Leu Trp Asn Trp Phe Ser Ile Thr Asn Trp Leu Trp Tyr Ile Lys
545                 550                 555                 560

Ile Phe Ile Met Ile Val Gly Gly Leu Ile Gly Leu Lys Ile Ile Phe
                565                 570                 575

Ala Val Leu Ser Ile Val Asn Arg Val Arg Gln Gly Tyr Ser Pro Leu
            580                 585                 590

Ser Phe Gln Thr
        595

(2) INFORMATION ZU SEQ ID NO: 3:

(i) SEQUENZ CHARAKTERISTIKA:
(A) LÄNGE: 1944 Basenpaare
(B) ART: Nukleinsäure
(C) STRANGFORM: nicht bekannt
(D) TOPOLOGIE: nicht bekannt

(ii) ART DES MOLEKÜLS: DNS (genomisch)

(vi) URSPRÜNLICHE HERKUNFT:
(A) ORGANISMUS: HIV-1D747

(ix) MERKMALE:
(A) NAME/SCHLÜSSEL: CDS
(B) LAGE: 131..1930

(xi) SEQUENZBESCHREIBUNG: SEQ ID NO: 3:

TAATGTCTGG GCAACACATG CCTGTGTACC CACAGACCCC AACCCACAAG AGATGGTTTT 60

GGGAAATGTA ACAGAAAATT TTAACATGTG GAGAAATGAC ATGGTGAATC AGATGCATGA 120

GGATGTAATC AGT TTA TGG GAT CAA AGC CTA AAG CCA TGT GTA AAG TTG 169
Ser Leu Trp Asp Gln Ser Leu Lys Pro Cys Val Lys Leu
1               5                   10

ACC CCA CTC TGT GTC ACT TTA CAT TGT ACA AAT GCT ACC TAT AGT AAT 217
Thr Pro Leu Cys Val Thr Leu His Cys Thr Asn Ala Thr Tyr Ser Asn
    15                  20                  25

AGT ACC TAC AAT AGT ACC TAC AAT GAA GAA ATA AAA AAT TGC TCT TTC 265
Ser Thr Tyr Asn Ser Thr Tyr Asn Glu Glu Ile Lys Asn Cys Ser Phe
30                  35                  40                  45

AAT ACA ACT ACG GAA CTA AGA GAT AAG AAA CAG AAA GTA CAA GCA CTT 313
Asn Thr Thr Thr Glu Leu Arg Asp Lys Lys Gln Lys Val Gln Ala Leu
                50                  55                  60

TTT TAT AAA CTT GAT GTA GTA CCA CTT AAT ACT ACT GAT AAC TCT AGT 361
Phe Tyr Lys Leu Asp Val Val Pro Leu Asn Thr Thr Asp Asn Ser Ser
            65                  70                  75

TAT AGA TTA ATA AAT TGT AAT ACC TCA GCC ATA ACA CAA GCC TGT CCA 409
Tyr Arg Leu Ile Asn Cys Asn Thr Ser Ala Ile Thr Gln Ala Cys Pro
        80                  85                  90

AAG GTC TCA TTT GAC CCA ATT CCT ATA CAT TAT TGT GCT GGA GCT GGT 457
Lys Val Ser Phe Asp Pro Ile Pro Ile His Tyr Cys Ala Gly Ala Gly
    95                  100                 105

TAT GCG ATT CTA AAG TGT AAT AAT AAG ACA TTC AAT GGG ACA GGA CCA 505
Tyr Ala Ile Leu Lys Cys Asn Asn Lys Thr Phe Asn Gly Thr Gly Pro
110                 115                 120                 125

TGC CAT AAT ATC AGC ACA GTA CAA TGT ACA CAT GGA ATT AAG CCA GTA 553
Cys His Asn Ile Ser Thr Val Gln Cys Thr His Gly Ile Lys Pro Val
                130                 135                 140

GTA TCA ACT CAA CTA CTG TTA AAT GGT AGC CTA GCA GAA GGA GAG ATA 601
Val Ser Thr Gln Leu Leu Leu Asn Gly Ser Leu Ala Glu Gly Glu Ile
            145                 150                 155

ATA ATT AGA TCT GAA AAT CTG ACA AAC AAT GTC AAA ACA ATA ATA GTA 649
Ile Ile Arg Ser Glu Asn Leu Thr Asn Asn Val Lys Thr Ile Ile Val
        160                 165                 170

CAT CTT AAT CAA TCT GTA GAA ATT GTG TAT ACA AGA CCC AAC AAT AAT 697
His Leu Asn Gln Ser Val Glu Ile Val Tyr Thr Arg Pro Asn Asn Asn
    175                 180                 185

ACA AGG AAA GGT GTA AGG ATA GGA CCA GGA CAA ACA TTC TAT GCA ACA 745
Thr Arg Lys Gly Val Arg Ile Gly Pro Gly Gln Thr Phe Tyr Ala Thr
190                 195                 200                 205

GGA GAC ATA ATA GGA GAC ATA AGA CAA GCA CAT TGT AAC ATT AGT AAA 793
Gly Asp Ile Ile Gly Asp Ile Arg Gln Ala His Cys Asn Ile Ser Lys
                210                 215                 220

CAT AAA TGG AAT GAA ACT TTA CAA AGG GTA GGT AAA AAA TTA GCA GAA 841
His Lys Trp Asn Glu Thr Leu Gln Arg Val Gly Lys Lys Leu Ala Glu
            225                 230                 235

CAC TTC CCT AAT AAA ACA ATA AGA TTT GCA TCA TCC TCA GGA GGG GAC 889
His Phe Pro Asn Lys Thr Ile Arg Phe Ala Ser Ser Ser Gly Gly Asp
        240                 245                 250

CTA GAA ATT ACA ACA CAT AGC TTT AAT TGT AGA GGA GAA TTT TTC TAT 937
Leu Glu Ile Thr Thr His Ser Phe Asn Cys Arg Gly Glu Phe Phe Tyr
    255                 260                 265

TGC AAT ACA TCA GAC CTG TTT AAT GGT ACA TAC ATG CCT AAT GGT ACA 985
Cys Asn Thr Ser Asp Leu Phe Asn Gly Thr Tyr Met Pro Asn Gly Thr
270                 275                 280                 285

AAA AGT AAT TCA AGC TCA ACC ATC ACA ATT CCA TGC AGA ATA AAA CAA 1033
Lys Ser Asn Ser Ser Ser Thr Ile Thr Ile Pro Cys Arg Ile Lys Gln
                290                 295                 300

ATT ATA AAC ATG TGG CAG GAG GTA GGA CGA GCA ATG TAT GCC CCT CCC 1081
Ile Ile Asn Met Trp Gln Glu Val Gly Arg Ala Met Tyr Ala Pro Pro
            305                 310                 315

ATT GCA GGA AAC ATA ACG TGT AAA TCC AAT ATT ACA GGA ATA CTA TTG 1129
Ile Ala Gly Asn Ile Thr Cys Lys Ser Asn Ile Thr Gly Ile Leu Leu
        320                 325                 330

GTA CGT GAT GGA GGA ATA GAG CTA AAT GAT ACA AAG ACA GAG ACA TTC 1177
Val Arg Asp Gly Gly Ile Glu Leu Asn Asp Thr Lys Thr Glu Thr Phe
    335                 340                 345

AGA CCG GGA GGA GGA GAA ATG AGG GAC AAT TGG AGA AGT GAA TTA TAT 1225
Arg Pro Gly Gly Gly Glu Met Arg Asp Asn Trp Arg Ser Glu Leu Tyr
350                 355                 360                 365

AAA TAT AAA GTG GTA GAA ATT AAG CCA TTG GGA GTA GCG CCC ACT ACT 1273
Lys Tyr Lys Val Val Glu Ile Lys Pro Leu Gly Val Ala Pro Thr Thr
                370                 375                 380

GCA AAA AGG AGA GTG GTG GAG AGA GAA AAA AGA GCA GTG GGA ATA GGA 1321
Ala Lys Arg Arg Val Val Glu Arg Glu Lys Arg Ala Val Gly Ile Gly
            385                 390                 395

GCT GTA TTC CTT GGG TTC TTG GGA GCA GCA GGA AGC ACT ATG GGC GCG 1369
Ala Val Phe Leu Gly Phe Leu Gly Ala Ala Gly Ser Thr Met Gly Ala
        400                 405                 410

GCG TCA ATG ACC GTG ACG GTA CAG GCC AGA CAA TTG TTG TCT GGT ATA 1417
Ala Ser Met Thr Val Thr Val Gln Ala Arg Gln Leu Leu Ser Gly Ile
    415                 420                 425

GTG CAA CAG CAA AGC AAT TTG CTG AGG GCT ATA GAG GCG CAA CAG CAT 1465
Val Gln Gln Gln Ser Asn Leu Leu Arg Ala Ile Glu Ala Gln Gln His
430                 435                 440                 445

CTG TTG CAA CTC ACG ATC TGG GGG ATT AAG CAG CTC CAG GCA AGA GTC 1513
Leu Leu Gln Leu Thr Ile Trp Gly Ile Lys Gln Leu Gln Ala Arg Val
                450                 455                 460

CTG GCT ATA GAA AGA TAC CTA AAG GAA CAA CAG CTC CTA GGG ATT TGG 1561
Leu Ala Ile Glu Arg Tyr Leu Lys Glu Gln Gln Leu Leu Gly Ile Trp
            465                 470                 475

GGC TGC TCT GGA AAA CTC ATC TGC ACC ACT ACT GTA CCT TGG AAC TCC 1609
Gly Cys Ser Gly Lys Leu Ile Cys Thr Thr Thr Val Pro Trp Asn Ser
        480                 485                 490

AGT TGG AGT AAC AGA TCT CAA ACA GAT ATT TGG GAT AAC ATG ACC TGG 1657
Ser Trp Ser Asn Arg Ser Gln Thr Asp Ile Trp Asp Asn Met Thr Trp
    495                 500                 505

ATG CAG TGG GAT AGA GAA ATT AGT AAT TAC ACA GAA ACA ATA TAC AGG 1705
Met Gln Trp Asp Arg Glu Ile Ser Asn Tyr Thr Glu Thr Ile Tyr Arg
510                 515                 520                 525

TTG CTT GAA GAC TCG CAA AAC CAG CAG GAA AGA AAT GAA AAA GAT TTA 1753
Leu Leu Glu Asp Ser Gln Asn Gln Gln Glu Arg Asn Glu Lys Asp Leu
                530                 535                 540

TTA GCA TTG GAC AGT TGG AAA AAT CTG TGG AAT TGG TTT AGC ATA ACA 1801
Leu Ala Leu Asp Ser Trp Lys Asn Leu Trp Asn Trp Phe Ser Ile Thr
            545                 550                 555

AAT TGG CTA TGG TAT ATA AAA ATA TTC ATA ATA ATA GTA GGA GGC TTG 1849
Asn Trp Leu Trp Tyr Ile Lys Ile Phe Ile Ile Ile Val Gly Gly Leu
        560                 565                 570

ATA GGC TTG AGA ATA ATT TTT GCT GTG CTT TGT ATA GTA AAT AGA GTT 1897
Ile Gly Leu Arg Ile Ile Phe Ala Val Leu Cys Ile Val Asn Arg Val
    575                 580                 585

AAG GCA GGA TAC TCA CCT TTG TCG TTT CAG ACC CTTACCCCGA ACCC 1944
Lys Ala Gly Tyr Ser Pro Leu Ser Phe Gln Thr
590                 595                 600

(2) INFORMATION ZU SEQ ID NO: 4:

(i) SEQUENZ CHARAKTERISTIKA:
(A) LÄNGE: 600 Aminosäuren
(B) ART: Aminosäure
(D) TOPOLOGIE: linear

(ii) ART DES MOLEKÜLS: Protein

(xi) SEQUENZBESCHREIBUNG: SEQ ID NO: 4:

Ser Leu Trp Asp Gln Ser Leu Lys Pro Cys Val Lys Leu Thr Pro Leu
1               5                   10                  15

Cys Val Thr Leu His Cys Thr Asn Ala Thr Tyr Ser Asn Ser Thr Tyr
            20                  25                  30

Asn Ser Thr Tyr Asn Glu Glu Ile Lys Asn Cys Ser Phe Asn Thr Thr
        35                  40                  45

Thr Glu Leu Arg Asp Lys Lys Gln Lys Val Gln Ala Leu Phe Tyr Lys
    50                  55                  60

Leu Asp Val Val Pro Leu Asn Thr Thr Asp Asn Ser Ser Tyr Arg Leu
65                  70                  75                  80

Ile Asn Cys Asn Thr Ser Ala Ile Thr Gln Ala Cys Pro Lys Val Ser
                85                  90                  95

Phe Asp Pro Ile Pro Ile His Tyr Cys Ala Gly Ala Gly Tyr Ala Ile
            100                 105                 110

Leu Lys Cys Asn Asn Lys Thr Phe Asn Gly Thr Gly Pro Cys His Asn
        115                 120                 125

Ile Ser Thr Val Gln Cys Thr His Gly Ile Lys Pro Val Val Ser Thr
    130                 135                 140

Gln Leu Leu Leu Asn Gly Ser Leu Ala Glu Gly Glu Ile Ile Ile Arg
145                 150                 155                 160

Ser Glu Asn Leu Thr Asn Asn Val Lys Thr Ile Ile Val His Leu Asn
                165                 170                 175

Gln Ser Val Glu Ile Val Tyr Thr Arg Pro Asn Asn Asn Thr Arg Lys
            180                 185                 190

Gly Val Arg Ile Gly Pro Gly Gln Thr Phe Tyr Ala Thr Gly Asp Ile
        195                 200                 205

Ile Gly Asp Ile Arg Gln Ala His Cys Asn Ile Ser Lys His Lys Trp
    210                 215                 220

Asn Glu Thr Leu Gln Arg Val Gly Lys Lys Leu Ala Glu His Phe Pro
225                 230                 235                 240

Asn Lys Thr Ile Arg Phe Ala Ser Ser Ser Gly Gly Asp Leu Glu Ile
                245                 250                 255

Thr Thr His Ser Phe Asn Cys Arg Gly Glu Phe Phe Tyr Cys Asn Thr
            260                 265                 270

Ser Asp Leu Phe Asn Gly Thr Tyr Met Pro Asn Gly Thr Lys Ser Asn
        275                 280                 285

Ser Ser Ser Thr Ile Thr Ile Pro Cys Arg Ile Lys Gln Ile Ile Asn
    290                 295                 300

Met Trp Gln Glu Val Gly Arg Ala Met Tyr Ala Pro Pro Ile Ala Gly
305                 310                 315                 320

Asn Ile Thr Cys Lys Ser Asn Ile Thr Gly Ile Leu Leu Val Arg Asp
                325                 330                 335

Gly Gly Ile Glu Leu Asn Asp Thr Lys Thr Glu Thr Phe Arg Pro Gly
            340                 345                 350

Gly Gly Glu Met Arg Asp Asn Trp Arg Ser Glu Leu Tyr Lys Tyr Lys
        355                 360                 365

Val Val Glu Ile Lys Pro Leu Gly Val Ala Pro Thr Thr Ala Lys Arg
    370                 375                 380

Arg Val Val Glu Arg Glu Lys Arg Ala Val Gly Ile Gly Ala Val Phe
385                 390                 395                 400

Leu Gly Phe Leu Gly Ala Ala Gly Ser Thr Met Gly Ala Ala Ser Met
                405                 410                 415

Thr Val Thr Val Gln Ala Arg Gln Leu Leu Ser Gly Ile Val Gln Gln
            420                 425                 430

Gln Ser Asn Leu Leu Arg Ala Ile Glu Ala Gln Gln His Leu Leu Gln
        435                 440                 445

Leu Thr Ile Trp Gly Ile Lys Gln Leu Gln Ala Arg Val Leu Ala Ile
    450                 455                 460

Glu Arg Tyr Leu Lys Glu Gln Gln Leu Leu Gly Ile Trp Gly Cys Ser
465                 470                 475                 480

Gly Lys Leu Ile Cys Thr Thr Thr Val Pro Trp Asn Ser Ser Trp Ser
                485                 490                 495

Asn Arg Ser Gln Thr Asp Ile Trp Asp Asn Met Thr Trp Met Gln Trp
            500                 505                 510

Asp Arg Glu Ile Ser Asn Tyr Thr Glu Thr Ile Tyr Arg Leu Leu Glu
        515                 520                 525

Asp Ser Gln Asn Gln Gln Glu Arg Asn Glu Lys Asp Leu Leu Ala Leu
    530                 535                 540

Asp Ser Trp Lys Asn Leu Trp Asn Trp Phe Ser Ile Thr Asn Trp Leu
545                 550                 555                 560

Trp Tyr Ile Lys Ile Phe Ile Ile Ile Val Gly Gly Leu Ile Gly Leu
                565                 570                 575

Arg Ile Ile Phe Ala Val Leu Cys Ile Val Asn Arg Val Lys Ala Gly
            580                 585                 590

Tyr Ser Pro Leu Ser Phe Gln Thr
        595                 600

(2) INFORMATION ZU SEQ ID NO: 5:

(i) SEQUENZ CHARAKTERISTIKA:
(A) LÄNGE: 1952 Basenpaare
(B) ART: Nukleinsäure
(C) STRANGFORM: nicht bekannt
(D) TOPOLOGIE: nicht bekannt

(ii) ART DES MOLEKÜLS: DNS (genomisch)

(vi) URSPRÜNLICHE HERKUNFT:
(A) ORGANISMUS: HIV-1D760

(ix) MERKMALE:
(A) NAME/SCHLÜSSEL: CDS
(B) LAGE: 133..1950

(xi) SEQUENZBESCHREIBUNG: SEQ ID NO: 5:

CATAATGTCT GGGCTACATA TGCCTGTGTA CCCACAGGCC CCGACCCACA AGAAATAGTT 60

TTGGAAAATG TAACAGGAAA TTTTAACATG TGGAAAAATG ACATGGTGGA TCAAATGCAT 120

GAGGATGTAA TC AGT TTA TGG GAT CAA AGC CTA AAG CCA TGT GTA AAG 168
Ser Leu Trp Asp Gln Ser Leu Lys Pro Cys Val Lys
1               5                   10

TTG ACC CCA CTC TGT GTC ACT TTA GAG TGT GGA AAT GTT AAT GCT ACC 216
Leu Thr Pro Leu Cys Val Thr Leu Glu Cys Gly Asn Val Asn Ala Thr
        15                  20                  25

AAT ATT ACC AAT AAT GGG GAA AAT AAT CCT ACC AAT ATT ACC AAT AAT 264
Asn Ile Thr Asn Asn Gly Glu Asn Asn Pro Thr Asn Ile Thr Asn Asn
    30                  35                  40

AGG GAA GAA ATA AAA AAT TGC CCT TTC AAT GCA ACC ACA GAA ATA AGA 312
Arg Glu Glu Ile Lys Asn Cys Pro Phe Asn Ala Thr Thr Glu Ile Arg
45                  50                  55                  60

GAT AGG CAG CAG AAA GTG TAT GCA CTT TTT TAT AGA CTT GAT ATA GTA 360
Asp Arg Gln Gln Lys Val Tyr Ala Leu Phe Tyr Arg Leu Asp Ile Val
                65                  70                  75

CCA CTT GAT AAT AAT AAT AAT AGC ACC TAT AGA TTA ATA AAT TGT AAT 408
Pro Leu Asp Asn Asn Asn Asn Ser Thr Tyr Arg Leu Ile Asn Cys Asn
            80                  85                  90

ACC TCA GCC ATA ACA CAA GCC TGT CCA AAG GTC ACT TTT GAT CCA ATT 456
Thr Ser Ala Ile Thr Gln Ala Cys Pro Lys Val Thr Phe Asp Pro Ile
        95                  100                 105

CCT ATA CAC TAT TGT GCT CCA GCT GGT TAT GCG ATT CTA AAG TGT AAT 504
Pro Ile His Tyr Cys Ala Pro Ala Gly Tyr Ala Ile Leu Lys Cys Asn
    110                 115                 120

AAT AAG ACA TTC AAT GGG ACA GGA CCA TGC CAT AAT GTC AGC ACA GTA 552
Asn Lys Thr Phe Asn Gly Thr Gly Pro Cys His Asn Val Ser Thr Val
125                 130                 135                 140

CAA TGT ACA CAT GGA ATT AGC CCA GTG GTA TCA ACT CAA CTA CTG TTA 600
Gln Cys Thr His Gly Ile Ser Pro Val Val Ser Thr Gln Leu Leu Leu
                145                 150                 155

AAT GGT AGC CTA GCA GAA GGA GAG ATA ATA ATT AGA TCT GAA AAT CTG 648
Asn Gly Ser Leu Ala Glu Gly Glu Ile Ile Ile Arg Ser Glu Asn Leu
            160                 165                 170

ACA GAC AAT GTC AAA ACA ATA ATA GTA CAT CTT AAT CAA TCT GTA GAA 696
Thr Asp Asn Val Lys Thr Ile Ile Val His Leu Asn Gln Ser Val Glu
        175                 180                 185

GTT GTG TGT ACA AGA CCC AAC AAT AAT ACA AGA AAA AGT ATA AGG ATA 744
Val Val Cys Thr Arg Pro Asn Asn Asn Thr Arg Lys Ser Ile Arg Ile
    190                 195                 200

GGA CCA GGA CAA ACA TTT TAT GCA ACA GGA GAC ATA ATA GGA GAC ATA 792
Gly Pro Gly Gln Thr Phe Tyr Ala Thr Gly Asp Ile Ile Gly Asp Ile
205                 210                 215                 220

AGA CAA GCA CAT TGT AAC ATT AGT GAA GAT AAA TGG AAT GAA ACT TTA 840
Arg Gln Ala His Cys Asn Ile Ser Glu Asp Lys Trp Asn Glu Thr Leu
                225                 230                 235

CAA AGG GTA GGT AAA AAA CTA GCA GAA CAC TTC CCT AAT AAA ACA ATA 888
Gln Arg Val Gly Lys Lys Leu Ala Glu His Phe Pro Asn Lys Thr Ile
            240                 245                 250

AAA TTT GCA GCA TCC TCA GGA GGG GAC CTA GAA ATT ACA ACA TAT AGT 936
Lys Phe Ala Ala Ser Ser Gly Gly Asp Leu Glu Ile Thr Thr Tyr Ser
        255                 260                 265

TTT AAT TGT AGA GGA GAA TTT TTC TAT TGC AAT ACA TCA GGC CTG TTC 984
Phe Asn Cys Arg Gly Glu Phe Phe Tyr Cys Asn Thr Ser Gly Leu Phe
    270                 275                 280

AAT GGT ACA TAC ATG CCT AAT GGT ACA AAA AGT AAT TTA AAC TCA ACC 1032
Asn Gly Thr Tyr Met Pro Asn Gly Thr Lys Ser Asn Leu Asn Ser Thr
285                 290                 295                 300

ATC ACA ATC CCA TGC AGA ATA AAA CAA ATT GTG AAC CTG TGG CAG GAG 1080
Ile Thr Ile Pro Cys Arg Ile Lys Gln Ile Val Asn Leu Trp Gln Glu
                305                 310                 315

GTA GGA CGA GCA ATG TAT GCC CCT CCA TTT GCC AGG AAC ATA ACA TGT 1128
Val Gly Arg Ala Met Tyr Ala Pro Pro Phe Ala Arg Asn Ile Thr Cys
            320                 325                 330

AAA TCA AAT ATC ACA GGA CTA CTA TTG GTA CGT GAT GGA GGA GAA GAC 1176
Lys Ser Asn Ile Thr Gly Leu Leu Leu Val Arg Asp Gly Gly Glu Asp
        335                 340                 345

ACA AAT GAT ACA GAG ATA TTC AGT CCT GGA GGA GGA GAT ATG AGG GAC 1224
Thr Asn Asp Thr Glu Ile Phe Ser Pro Gly Gly Gly Asp Met Arg Asp
    350                 355                 360

AAT TGG AGA AGT GAA TTA TAC AAA TAT AAA GTG GTA GAA ATT AAG CCA 1272
Asn Trp Arg Ser Glu Leu Tyr Lys Tyr Lys Val Val Glu Ile Lys Pro
365                 370                 375                 380

TTG GGA GTA GCA CCC ACT ACA GCA AAA AGG AGA GTG GTG GAG AGA GAA 1320
Leu Gly Val Ala Pro Thr Thr Ala Lys Arg Arg Val Val Glu Arg Glu
                385                 390                 395

AAA AGA GCA GTG GGA TTA GGA GCT GTG TTC CTT GGG TTC TTG GGA GCA 1368
Lys Arg Ala Val Gly Leu Gly Ala Val Phe Leu Gly Phe Leu Gly Ala
            400                 405                 410

GCA GGA AGC ACT ATG GGC GCG GCG TCA ATA ACG CTG ACG GTA CAG GCC 1416
Ala Gly Ser Thr Met Gly Ala Ala Ser Ile Thr Leu Thr Val Gln Ala
        415                 420                 425

AGA CAA TTA CTG TCT GGT ATA GTG CAA CAG CAA AGC AAT TTG CTG AGG 1464
Arg Gln Leu Leu Ser Gly Ile Val Gln Gln Gln Ser Asn Leu Leu Arg
    430                 435                 440

GCT ATA GAG GCG CAA CAG CAT CTG TTG CAA CTC ACG GTC TGG GGC ATT 1512
Ala Ile Glu Ala Gln Gln His Leu Leu Gln Leu Thr Val Trp Gly Ile
445                 450                 455                 460

AAG CAG CTC CAG ACA AGA GTC CTG GCT ATA GAA AGA TAC CTA AAG GAT 1560
Lys Gln Leu Gln Thr Arg Val Leu Ala Ile Glu Arg Tyr Leu Lys Asp
                465                 470                 475

CAA CAG CTC CTA GGG ATG TGG GGC TGC TCT GGA AAA CTC ATC TGC ACC 1608
Gln Gln Leu Leu Gly Met Trp Gly Cys Ser Gly Lys Leu Ile Cys Thr
            480                 485                 490

ACT GCT GTA CCT TGG AAC TCC AGT TGG AGT AAC AGA TCT CAA ACA GAT 1656
Thr Ala Val Pro Trp Asn Ser Ser Trp Ser Asn Arg Ser Gln Thr Asp
        495                 500                 505

ATT TGG GAT AAC ATG ACC TGG ATG CAG TGG GAT AGG GAA ATT AGT AAT 1704
Ile Trp Asp Asn Met Thr Trp Met Gln Trp Asp Arg Glu Ile Ser Asn
    510                 515                 520

TAC ACA AAT ACA ATA TAC AGG TTG CTT GAA GAC TCG CAA AAC CAG CAG 1752
Tyr Thr Asn Thr Ile Tyr Arg Leu Leu Glu Asp Ser Gln Asn Gln Gln
525                 530                 535                 540

GAA AGA AAT GAA AAA GAT TTA TTA GCA TTG GAC AGT TGG AAA AAT CTG 1800
Glu Arg Asn Glu Lys Asp Leu Leu Ala Leu Asp Ser Trp Lys Asn Leu
                545                 550                 555

TGG AAT TGG TTT AGC ATA ACA AAT TGG CTG TGG TAT ATA AAA ATA TTC 1848
Trp Asn Trp Phe Ser Ile Thr Asn Trp Leu Trp Tyr Ile Lys Ile Phe
            560                 565                 570

ATA ATG ATA GTA GGA GGC TTG ATA GGT TTG AGA ATA ATT TTT GCT GTG 1896
Ile Met Ile Val Gly Gly Leu Ile Gly Leu Arg Ile Ile Phe Ala Val
        575                 580                 585

CTC TCT ATA GTG AAT AGA GTT AGG CAG GGA TAC TCA CCT TTG TCG TTT 1944
Leu Ser Ile Val Asn Arg Val Arg Gln Gly Tyr Ser Pro Leu Ser Phe
    590                 595                 600

CAG ACC CT 1952
Gln Thr
605

(2) INFORMATION ZU SEQ ID NO: 6:

(i) SEQUENZ CHARAKTERISTIKA:
(A) LÄNGE: 606 Aminosäuren
(B) ART: Aminosäure
(D) TOPOLOGIE: linear

(ii) ART DES MOLEKÜLS: Protein

(xi) SEQUENZBESCHREIBUNG: SEQ ID NO: 6:

Ser Leu Trp Asp Gln Ser Leu Lys Pro Cys Val Lys Leu Thr Pro Leu
1               5                   10                  15

Cys Val Thr Leu Glu Cys Gly Asn Val Asn Ala Thr Asn Ile Thr Asn
            20                  25                  30

Asn Gly Glu Asn Asn Pro Thr Asn Ile Thr Asn Asn Arg Glu Glu Ile
        35                  40                  45

Lys Asn Cys Pro Phe Asn Ala Thr Thr Glu Ile Arg Asp Arg Gln Gln
    50                  55                  60

Lys Val Tyr Ala Leu Phe Tyr Arg Leu Asp Ile Val Pro Leu Asp Asn
65                  70                  75                  80

Asn Asn Asn Ser Thr Tyr Arg Leu Ile Asn Cys Asn Thr Ser Ala Ile
                85                  90                  95

Thr Gln Ala Cys Pro Lys Val Thr Phe Asp Pro Ile Pro Ile His Tyr
            100                 105                 110

Cys Ala Pro Ala Gly Tyr Ala Ile Leu Lys Cys Asn Asn Lys Thr Phe
        115                 120                 125

Asn Gly Thr Gly Pro Cys His Asn Val Ser Thr Val Gln Cys Thr His
    130                 135                 140

Gly Ile Ser Pro Val Val Ser Thr Gln Leu Leu Leu Asn Gly Ser Leu
145                 150                 155                 160

Ala Glu Gly Glu Ile Ile Ile Arg Ser Glu Asn Leu Thr Asp Asn Val
                165                 170                 175

Lys Thr Ile Ile Val His Leu Asn Gln Ser Val Glu Val Val Cys Thr
            180                 185                 190

Arg Pro Asn Asn Asn Thr Arg Lys Ser Ile Arg Ile Gly Pro Gly Gln
        195                 200                 205

Thr Phe Tyr Ala Thr Gly Asp Ile Ile Gly Asp Ile Arg Gln Ala His
    210                 215                 220

Cys Asn Ile Ser Glu Asp Lys Trp Asn Glu Thr Leu Gln Arg Val Gly
225                 230                 235                 240

Lys Lys Leu Ala Glu His Phe Pro Asn Lys Thr Ile Lys Phe Ala Ala
                245                 250                 255

Ser Ser Gly Gly Asp Leu Glu Ile Thr Thr Tyr Ser Phe Asn Cys Arg
            260                 265                 270

Gly Glu Phe Phe Tyr Cys Asn Thr Ser Gly Leu Phe Asn Gly Thr Tyr
        275                 280                 285

Met Pro Asn Gly Thr Lys Ser Asn Leu Asn Ser Thr Ile Thr Ile Pro
    290                 295                 300

Cys Arg Ile Lys Gln Ile Val Asn Leu Trp Gln Glu Val Gly Arg Ala
305                 310                 315                 320

Met Tyr Ala Pro Pro Phe Ala Arg Asn Ile Thr Cys Lys Ser Asn Ile
                325                 330                 335

Thr Gly Leu Leu Leu Val Arg Asp Gly Gly Glu Asp Thr Asn Asp Thr
            340                 345                 350

Glu Ile Phe Ser Pro Gly Gly Gly Asp Met Arg Asp Asn Trp Arg Ser
        355                 360                 365

Glu Leu Tyr Lys Tyr Lys Val Val Glu Ile Lys Pro Leu Gly Val Ala
    370                 375                 380

Pro Thr Thr Ala Lys Arg Arg Val Val Glu Arg Glu Lys Arg Ala Val
385                 390                 395                 400

Gly Leu Gly Ala Val Phe Leu Gly Phe Leu Gly Ala Ala Gly Ser Thr
                405                 410                 415

Met Gly Ala Ala Ser Ile Thr Leu Thr Val Gln Ala Arg Gln Leu Leu
            420                 425                 430

Ser Gly Ile Val Gln Gln Gln Ser Asn Leu Leu Arg Ala Ile Glu Ala
        435                 440                 445

Gln Gln His Leu Leu Gln Leu Thr Val Trp Gly Ile Lys Gln Leu Gln
    450                 455                 460

Thr Arg Val Leu Ala Ile Glu Arg Tyr Leu Lys Asp Gln Gln Leu Leu
465                 470                 475                 480

Gly Met Trp Gly Cys Ser Gly Lys Leu Ile Cys Thr Thr Ala Val Pro
                485                 490                 495

Trp Asn Ser Ser Trp Ser Asn Arg Ser Gln Thr Asp Ile Trp Asp Asn
            500                 505                 510

Met Thr Trp Met Gln Trp Asp Arg Glu Ile Ser Asn Tyr Thr Asn Thr
        515                 520                 525

Ile Tyr Arg Leu Leu Glu Asp Ser Gln Asn Gln Gln Glu Arg Asn Glu
    530                 535                 540

Lys Asp Leu Leu Ala Leu Asp Ser Trp Lys Asn Leu Trp Asn Trp Phe
545                 550                 555                 560

Ser Ile Thr Asn Trp Leu Trp Tyr Ile Lys Ile Phe Ile Met Ile Val
                565                 570                 575

Gly Gly Leu Ile Gly Leu Arg Ile Ile Phe Ala Val Leu Ser Ile Val
            580                 585                 590

Asn Arg Val Arg Gln Gly Tyr Ser Pro Leu Ser Phe Gln Thr
        595                 600                 605

(2) INFORMATION ZU SEQ ID NO: 7:

(i) SEQUENZ CHARAKTERISTIKA:
(A) LÄNGE: 9 Aminosäuren
(B) ART: Aminosäure
(D) TOPOLOGIE: nicht bekannt

(ii) ART DES MOLEKÜLS: Peptid

(xi) SEQUENZBESCHREIBUNG: SEQ ID NO: 7:

Met Pro Asn Gly Thr Lys Ser Asn Ser
1               5

(2) INFORMATION ZU SEQ ID NO: 8:

(i) SEQUENZ CHARAKTERISTIKA:
(A) LÄNGE: 9 Aminosäuren
(B) ART: Aminosäure
(D) TOPOLOGIE: nicht bekannt

(ii) ART DES MOLEKÜLS: Peptid

(xi) SEQUENZBESCHREIBUNG: SEQ ID NO: 8:

Met Pro Asn Gly Thr Lys Gly Asn Ser
1               5

(2) INFORMATION ZU SEQ ID NO: 9:

(i) SEQUENZ CHARAKTERISTIKA:
(A) LÄNGE: 9 Aminosäuren
(B) ART: Aminosäure
(D) TOPOLOGIE: nicht bekannt

(ii) ART DES MOLEKÜLS: Peptid

(xi) SEQUENZBESCHREIBUNG: SEQ ID NO: 9:

Met Pro Asn Gly Thr Lys Ser Asn Leu
1               5

(2) INFORMATION ZU SEQ ID NO: 10:

(i) SEQUENZ CHARAKTERISTIKA:
(A) LÄNGE: 14 Aminosäuren
(B) ART: Aminosäure
(D) TOPOLOGIE: nicht bekannt

(ii) ART DES MOLEKÜLS: Peptid

(xi) SEQUENZBESCHREIBUNG: SEQ ID NO: 10:

Arg Asn Glu Lys Asp Leu Leu Ala Leu Asp Ser Trp Lys Asn
1               5                   10